(19) 

Europäisches
Patentamt
European
Patent Office
Office européen
des brevets

(11) **EP 2 671 590 A1**

(12) **EUROPEAN PATENT APPLICATION**
published in accordance with Art. 153(4) EPC

(43) Date of publication:
**11.12.2013 Bulletin 2013/50**

(21) Application number: **12741901.8**

(22) Date of filing: **30.01.2012**

(51) Int Cl.:
*A61K 31/513* (2006.01)    *A61K 31/4412* (2006.01)
*A61K 31/53* (2006.01)    *A61P 35/00* (2006.01)
*A61P 43/00* (2006.01)

(86) International application number:
**PCT/JP2012/051970**

(87) International publication number:
**WO 2012/105486 (09.08.2012 Gazette 2012/32)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **31.01.2011 JP 2011018616**

(71) Applicant: **Taiho Pharmaceutical Co., Ltd.
Chiyoda-ku, Tokyo 101-8444 (JP)**

(72) Inventors:
• **YAMAUE, Hiroki
Wakayama-shi
Wakayama 640-8472 (JP)**

• **YAMAMITSU, Susumu
Sapporo-shi
Hokkaido 062-0051 (JP)**
• **SHIRASAKA, Tetsuhiko
Saitama 3570041 (JP)**

(74) Representative: **HOFFMANN EITLE
Patent- und Rechtsanwälte
Arabellastrasse 4
81925 München (DE)**

(54) **TEGAFUR-CONTAINING COMPOSITION FOR SINGLE DAILY AND ALTERNATE-DAY DOSING**

(57)    This invention provides an antitumor agent comprising a combination drug containing tegafur, gimeracil, and oteracil potassium, characterized by being repeatedly administered to a patient once a day every other day, four days a week, at a dose such that the tegafur is administered in an amount of 80 mg or more/dose when the patient has a body surface area of less than 1.25 m$^2$, in an amount of 100 mg or more/dose when the patient has a body surface area of 1.25 to 1.5 m$^2$, or in an amount of 120 mg or more/dose when the patient has a body surface area of not less than 1.5 m$^2$.

EP 2 671 590 A1

**Description**

Technical Field

This application claims priority to Japanese Patent Application No. 2011-018616, filed on January 31, 2011, the entire contents of which are hereby incorporated by reference.

The present invention relates to an antitumor agent; more specifically, the present invention relates to an antitumor agent comprising a combination drug containing tegafur, gimeracil, and oteracil potassium that is repeatedly administered once a day every other day, four days a week; a method for treating cancer; and a use of the combination drug.

Background Art

A combination drug containing tegafur, gimeracil, and oteracil potassium is an antitumor agent in which tegafur, which is a prodrug of fluorouracil (5-FU), is combined with gimeracil, which is an inhibitor of degradation of 5-FU, and oteracil potassium, which is an inhibitor of phosphorylation of 5-FU, thereby reducing gastrointestinal toxicities while enhancing an antitumor effect. The combination drug is widely used in clinical settings as a cancer chemotherapeutic agent that can be orally administered (Patent Literature 1).

Currently, a combination drug containing tegafur, gimeracil, and oteracil potassium, in such a manner that the molar ratio of tegafur:gimeracil:oteracil potassium is 1:0.4:1, is sold under the names "TS-1 combination capsule" and "TS-1 combination granule" (Taiho Pharmaceutical Co., Ltd.). In Japan, the combination drug has efficacy against a broad range of cancers such as stomach cancer, colorectal cancer, head and neck cancer, non-small cell lung cancer, inoperable or recurrent breast cancer, pancreatic cancer, and biliary tract cancer. The dosage regimen of the combination drug is described in the package insert as follows. For adults, a standard dose according to body surface area is usually defined as an initial dose (single dose). The combination drug is orally administered twice a day, after breakfast and after dinner, for 28 consecutive days, followed by a 14-day withdrawal. This is defined as one course, and administration is repeated. As the single dose, the following standard dose is defined according to body surface area: the amount of tegafur is 40 mg/dose for a body surface area of less than 1.25 $m^2$, 50 mg/dose for a body surface area of 1.25 to 1.5 $m^2$, and 60 mg/dose for a body surface area of not less than 1.5 $m^2$. These doses may be suitably increased or decreased depending on the patient's condition. When there are no abnormalities in laboratory findings (blood test and liver and renal function tests) attributable to administration of the combination drug, no gastrointestinal symptoms, and no problems in regards to safety; and, if it is determined that the dose may be increased, the initial doses may be only increased by one stage, i.e., increased individually to 50 mg/dose, 60 mg/dose, and 75 mg/dose, according to the above body surface area.

About 13 years have passed since TS-1 was approved, and the side effects thereof have been reported in detail. In the above-described standard dosage regimen of TS-1 alone in which TS-1 is administered twice a day, for 28 consecutive days, followed by a 14-day withdrawal, many patients suffer primarily from nonhematological toxic side effects, which makes it difficult to receive continuous long-term treatment. Thus, various therapeutic methods have also been explored in clinical settings.

In recent years, a method for administering TS-1 twice a day every other day has been proposed as a therapeutic method to solve the essential problem of reducing patients' physical discomfort, and it is reported that side effects can be reduced (e.g., Non-patent Literature 1 to 3). On the other hand, a method for repeatedly administering TS-1 once a day every other day, three days a week is also reported; however, it is disclosed that the incidence of adverse events was high, and that the response rate was low (Non-patent Literature 4).

As described above, there is a need to establish a novel therapeutic method and a novel method of preventing recurrence that further reduce side effects, in particular, incidence of nonhematological toxicities, thereby contributing to more cancer patients and allowing continuous long-term treatment.

Citation List

Patent Literature

PTL 1: JP2614164B

Non-patent Literature

NPL 1: Int J Clin Oncol (2008) 13:515-520
NPL 2: Int J Clin Oncol (2010) 15:166-171
NPL 3: The Japanese journal of gastroenterological surgery, Vol. 39 (4), p. 486 (2006)
NPL 4: Nihon gan chiryo gakkaishi [The Journal of Japan Society of Clinical Oncology], Vol. 38, no. 2, p. 518 (2003)

Summary of Invention

Technical Problem

An object of the present invention is to provide a novel method of using a combination drug containing tegafur, gimeracil, and oteracil potassium that provides a higher therapeutic effect by reducing incidence and severity of side effects such as hematological and nonhematological toxicities, thereby allowing continuous long-term treatment.

Solution to Problem

The present inventors conducted extensive research on a method of using a combination drug containing tegafur, gimeracil, and oteracil potassium, and found that when it is repeatedly administered to a cancer patient once a day every other day, four days a week, at a dose such that the tegafur is administered in an amount of 80 mg or more/ dose when the patient has a body surface area of less than 1.25 $m^2$, in an amount of 100 mg or more/dose when the patient has a body surface area of 1.25 to 1.5 $m^2$, or in an amount of 120 mg or more/dose when the patient has a body surface area of not less than 1.5 $m^2$, incidence and severity of side effects are significantly reduced, thereby allowing continuous long-term treatment, resulting in a high antitumor effect; and that this is an effective method that contributes to the prolongation of survival time and quality of life (QOL) of patients.

Specifically, the present invention relates to the following 1) to 12).

1) An antitumor agent comprising a combination drug containing tegafur, gimeracil, and oteracil potassium, characterized by being repeatedly administered to a patient once a day every other day, four days a week, at a dose such that the tegafur is administered in an amount of 80 mg or more/dose when the patient has a body surface area of less than 1.25 $m^2$, in an amount of 100 mg or more/dose when the patient has a body surface area of 1.25 to 1.5 $m^2$, or in an amount of 120 mg or more/dose when the patient has a body surface area of not less than 1.5 $m^2$.

2) An antitumor agent comprising a combination drug containing tegafur, gimeracil, and oteracil potassium, characterized by being repeatedly administered to a patient once a day every other day, four days a week, at a dose such that the tegafur is administered in an amount of 100 mg or more/dose when the patient has a body surface area of less than 1.25 $m^2$, in an amount of 120 mg or more/dose when the patient has a body surface area of 1.25 to 1.5 $m^2$, or 150 mg or more/dose when the patient has a body surface area of not less than 1.5 $m^2$.

3) The antitumor agent comprising a combination drug containing tegafur, gimeracil, and oteracil potassium according to 1) or 2), which gives the maximum blood 5-FU concentration of 180 to 450 ng/ml, and a blood 5-FU concentration of 10 ng/ml or lower within 24 hours after administration, in the patient administered the combination drug containing tegafur, gimeracil, and oteracil potassium.

4) The antitumor agent comprising a combination drug containing tegafur, gimeracil, and oteracil potassium according to 1) to 3), which gives the maximum blood 5-FU concentration of 180 to 450 ng/ml within 4 hours after administration and a blood 5-FU concentration of 10 ng/ml or lower within 24 hours after administration, in the patient administered the combination drug containing tegafur, gimeracil, and oteracil potassium.

5) The antitumor agent according to 1) to 4), wherein the molar ratio of respective active ingredients in the combination drug containing tegafur, gimeracil, and oteracil potassium, i.e., the molar ratio of tegafur:gimeracil:oteracil potassium, is 1:0.4:1.

6) The antitumor agent according to 1) to 5), wherein the combination drug is repeatedly administered every Monday, every Wednesday, every Friday, and every Sunday.

7) A method for treating cancer, comprising  repeatedly administering a combination drug containing tegafur, gimeracil, and oteracil potassium to a patient once a day every other day, four days a week, at a dose such that the tegafur is administered in an amount of 80 mg or more/dose when the patient has a body surface area of less than 1.25 $m^2$, in an amount of 100 mg or more/dose when the patient has a body surface area of 1.25 to 1.5 $m^2$, or in an amount of 120 mg or more/dose when the patient has a body surface area of not less than 1.5 $m^2$.

8) A method for treating cancer, comprising repeatedly administering a combination drug containing tegafur, gimeracil, and oteracil potassium to a patient once a day every other day, four days a week, at a dose such that the tegafur is administered in an amount of 100 mg or more/dose when the patient has a body surface area of less than 1.25 m$^2$, in an amount of 120 mg or more/dose when the patient has a body surface area of 1.25 to 1.5 m$^2$, or in an amount of 150 mg or more/dose when the patient has a body surface area of not less than 1.5 m$^2$.

9) Use of a combination drug containing tegafur, gimeracil, and oteracil potassium for the production of a medicament for treating cancer, the combination drug being repeatedly administered to a patient once a day every other day, four days a week, at a dose such that the tegafur is administered in an amount of 80 mg or more/dose when the patient has a body surface area of less than 1.25 m$^2$, in an amount of 100 mg or more/dose when the patient has a body surface area of 1.25 to 1.5 m$^2$, or in an amount of 120 mg or more/dose when the patient has a body surface area of not less than 1.5 m$^2$.

10) Use of a combination drug containing tegafur, gimeracil, and oteracil potassium for the production of a medicament for treating cancer, the combination drug being repeatedly administered to a patient once a day every other day, four days a week, at a dose such that the tegafur is administered  in an amount of 100 mg or more/dose when the patient has a body surface area of less than 1.25 m$^2$, in an amount of 120 mg or more/dose when the patient has a body surface area of 1.25 to 1.5 m$^2$, or in an amount of 150 mg or more/dose when the patient has a body surface area of not less than 1.5 m$^2$.

11) A combination drug containing tegafur, gimeracil, and oteracil potassium for use in the treatment of cancer, the combination drug being repeatedly administered to a patient once a day every other day, four days a week, at a dose such that the tegafur is administered in an amount of 80 mg or more/dose when the patient has a body surface area of less than 1.25 m$^2$, in an amount of 100 mg or more/dose when the patient has a body surface area of 1.25 to 1.5 m$^2$, or in an amount of 120 mg or more/dose when the patient has a body surface area of not less than 1.5 m$^2$.

12) A combination drug containing tegafur, gimeracil, and oteracil potassium for use in the treatment of cancer, the combination drug being repeatedly administered to a patient once a day every other day, four days a week, at a dose such that the tegafur is administered in an amount of 100 mg or more/dose when the patient has a body surface area of less than 1.25 m$^2$, in an amount of 120 mg or more/dose when the patient has a body surface area of 1.25 to 1.5 m$^2$, or in an amount of 150 mg or more/dose when the patient has a body surface area of not less than 1.5 m$^2$.

Advantageous Effects of Invention

According to the novel method of using a combination drug containing tegafur, gimeracil, and oteracil potassium of the present invention, a therapeutic method having a higher therapeutic effect can be provided by reducing the incidence and severity of side effects such as hematological and nonhematological toxicities, thereby allowing continuous long-term treatment.

When administration is performed at the same daily dose as described in the current package insert for 6 weeks in the administration method of the present invention, in which the antitumor agent is administered every other day, four times a week, the total amount that is administered for 6 weeks in the present invention is less than that in the standard dosage regimen, in which administration is performed twice a day for 4 weeks, followed by a 2-week withdrawal. Both the antitumor effect and side effects are expected to decrease when the total amount that is administered is low; however, it is revealed that the antitumor agent of the present invention provides an antitumor effect that is equal to or better than that of conventional administration methods, and significantly reduces the incidence and severity of side effects. When the daily dose (tegafur amount) is increased, for example, from 80 mg to 100 mg, from 100 mg to 120 mg, from 120 mg to 150 mg, or from 150 mg to 180 mg, the total amount that is administered for 6 weeks is slightly higher than that in the standard dosage regimen. In this case, despite the higher total amount that is administered, the antitumor agent of the present invention is expected to significantly reduce the incidence and severity of side effects, and provide an antitumor effect superior to that of conventional administration methods.

When the daily dose of the antitumor agent of the present invention is the same daily dose as the dosage regimen described in the package insert, which is a combination of 4-week twice-a-day administration and 2-week withdrawal, the antitumor agent of the present invention can achieve both reduction in side effects and improvement in efficacy. The antitumor agent of the present invention, which has significantly reduced incidence and severity of side effects, can be administered for a long period of time in adjuvant chemotherapy for preventing recurrence, and more potent efficacy can be expected by increasing the dose.

The antitumor agent of the present invention comprises a combination drug containing tegafur, gimeracil, and oteracil potassium as an active ingredient. While not wishing to be bound by theory, it is believed that unlike the case of single active ingredient preparations, the present invention, which comprises the combination drug as an active ingredient, achieves unexpected effects, i.e., achieves both improvement in efficacy, and reduction in incidence and severity of side effects by changing the administration method.

Brief Description of Drawings

Fig. 1 shows the survival rates and survival times of mice obtained in Example 1.
Fig. 2 shows the body weight changes of mice obtained in Example 1.
Fig. 3 shows change in blood concentration of 5-FU in Subject 1 of Example 2 (at the time of administration in a tegafur amount of 100 mg).
Fig. 4 shows change in blood concentration of 5-FU in Subject 2 of Example 2 (at the time of administration in a tegafur amount of 80 mg).
Fig. 5 shows change in blood concentration of 5-FU in Subject 2 of Example 2 (at the time of administration in a tegafur amount of 120 mg).
Fig. 6 shows change in blood concentration of 5-FU in Subject 3 of Example 2 (at the time of administration in a tegafur amount of 100 mg).
Fig. 7 shows change in blood concentration of 5-FU in Subject 4 of Example 2 (at the time of administration in a tegafur amount of 120 mg).
Fig. 8 shows change in blood concentration of 5-FU in Subject 5 of Example 2 (at the time of administration in a tegafur amount of 120 mg).
Fig. 9 shows change in blood concentration of 5-FU in Subject 5 of Example 2 (at the time of administration in a tegafur amount of 150 mg).
Fig. 10 shows change in blood concentration of 5-FU in Subject 6 of Example 2 (at the time of administration in a tegafur amount of 120 mg).

Description of Embodiments

An administration method for a currently used combination drug containing tegafur, gimeracil, and oteracil potassium is described in the package insert as follows. The combination drug containing tegafur, gimeracil, and oteracil potassium is orally administered twice a day, after breakfast and after dinner, for 28 consecutive days, followed by a 14-day withdrawal. This is defined as one course, and administration is repeated. This administration method is regarded as a standard dosage regimen. As the single dose, the following standard dose is defined according to body surface area: the amount of tegafur is 40 mg/dose for a body surface area of less than 1.25 $m^2$, 50 mg/dose for a body surface area of 1.25 to 1.5 $m^2$, and 60 mg/dose for a body surface area of not less than 1.5 $m^2$. These doses may be suitably increased or decreased depending on the patient's condition. The dose may be increased to 50 mg/dose when the standard dose is 40 mg/dose; 60 mg/dose when the standard dose is 50 mg/dose; and 75 mg/dose when the standard dose is 60 mg. Since it is described that the dose can be only increased by one stage, the upper limit of a single dose in the standard dosage regimen is 75 mg/dose.

On the other hand, a method for administering the combination drug containing tegafur, gimeracil, and oteracil potassium twice a day, every other day has been recently proposed as a therapeutic method with fewer side effects of the combination drug. Non-patent Literature 1 discloses an administration group given TS-1 every day and an administration group given TS-1 every other day in a basic study. More specifically, it is reported that the administration group given TS-1 every other day had a relative inhibition period of cancer growth equivalent to that of the administration group given TS-1 every day, and the every-other-day administration had low toxicities. Non-patent Literature 2 discloses clinical results of 266 patients with stomach cancer in a method for administering TS-1 twice a day every other day, and reports that side effects can be reduced without impairing a therapeutic effect. Non-patent Literature 3 discloses a method for orally administering TS-1 twice a day every other day in a dose of 50 mg at 3 p.m., and a dose of 100 mg at 10 p.m. for postoperative multiple liver metastatic foci from ampullary carcinoma; and reports that the method had no side effects and allowed out-patient medical treatment.

Regarding a method for administering TS-1 once a day every other day, Non-patent Literature 4 discloses a method for administering TS-1 to patients with advanced/recurrent stomach cancer once a day every other day, three times a week, i.e., Monday, Wednesday, and Friday, in a dose of 80 mg, which is twice the standard dose; and reports that the incidence of adverse events was high and the response rate was low.

As described above, a method for repeatedly administering the combination drug containing tegafur, gimeracil, and oteracil potassium once a day every other day, four days a week is nowhere disclosed. In view of concerns such as onset of side effects as disclosed in Non-patent Literature 4, a method for repeatedly administering the combination drug containing tegafur, gimeracil, and oteracil potassium once a day every other day, four days a week in an amount that is twice or more the single dose of the standard dosage regimen in which administration is performed twice a day is an administration method that is not obvious from the prior art.

The present inventors, however, found that a method for repeatedly administering the combination drug containing tegafur, gimeracil, and oteracil potassium to a patient once a day every other day, four days a week at a dose that is twice the single dose defined in the package insert as the standard dose in terms of tegafur amount per body surface area (i.e., at a dose such that the tegafur is administered in an amount of 80 mg/dose when the patient has a body surface area of less than 1.25 m$^2$, in an amount of 100 mg/dose when the patient has a body surface area of 1.25 to 1.5 m$^2$, or in an amount of 120 mg/dose when the patient has a body surface area of not less than 1.5 m$^2$) or at a greater dose is an administration method that has, contrary to expectations, less incidence and severity of side effects, and higher therapeutic effect despite one more administration day per week compared to Non-patent Literature 4.

According to the present invention, an antitumor effect can be further enhanced compared to the twice-a-day administration since the blood concentration of 5-FU is rapidly increased by administering a high dose of the combination drug once a day, while side effects caused by conventional TS-1 administration methods can be avoided since 5-FU nearly disappears from blood within 24 hours after administration and administration is performed every other day. More specifically, the antitumor agent of the present invention makes it possible to achieve both high antitumor effect and reduction in side effects.

In addition, the patients' convenience is also improved compared to when administering the combination drug containing tegafur, gimeracil, and oteracil potassium twice a day every other day, since patients only take the combination drug containing tegafur, gimeracil, and oteracil potassium once a day.

The phrase "repeatedly administered every other day, four days a week" means that four days of the week are selected such that days when the combination drug containing tegafur, gimeracil, and oteracil potassium is not administered are non-successive, and the combination drug containing tegafur, gimeracil, and oteracil potassium is repeatedly administered on the selected days every week. More specifically, four days a week means, but is not limited to, four days: Monday, Wednesday, Friday, and Sunday; four days: Monday, Tuesday, Thursday, and Saturday; four days: Monday, Wednesday, Friday, and Saturday; or the like.

In the present specification, the "antitumor agent" is useful for treating cancer or tumor, and/or preventing recurrence of cancer or tumor. Thus, the present invention provides an agent for treating cancer or tumor, and an agent for preventing recurrence of cancer or tumor. Here, preventing recurrence means preventing recurrence of cancer or tumor after cancer or tumor tissues disappear once or cannot be found once following surgery, radiotherapy, chemotherapy, or the like. For preventing recurrence of cancer or tumor, the combination drug is repeatedly administered to a patient once a day every other day, four days a week, at a dose such that the tegafur is administered in an amount of 80 mg or more/dose when the patient has a body surface area of less than 1.25 m$^2$, in an amount of 100 mg or more/dose when the patient has a body surface area of 1.25 to 1.5 m$^2$, or in an amount of 120 mg or more/dose when the patient has a body surface area of not less than 1.5 m$^2$; or repeatedly administered to a patient once a day every other day, four days a week, at a dose such that the tegafur is administered in an amount of 100 mg or more/dose when the patient has a body surface area of less than 1.25 m$^2$, in an amount of 120 mg or more/dose when the patient has a body surface area of 1.25 to 1.5 m$^2$, or in an amount of 150 mg or more/dose when the patient has a body surface area of not less than 1.5 m$^2$. The administration period for preventing recurrence is usually about 1 month to about 1 year, in particular, about 3 months to about 6 months. Recurrence of cancer or tumor can be prevented when the antitumor agent is continually administered during the period.

Since the antitumor agent of the present invention is administered once a day, the single dose is larger and the blood concentration of 5-FU, which is the active ingredient, is higher compared to those in the twice-a-day administration. When administered once a day at the daily dose according to body surface area described in the package insert, the maximum blood concentration of 5-FU, although it varies depending on the individual, is believed to be within the range of about 180 to about 450 ng/ml. The blood concentration can increase as the daily dose increases.

Examples of the dose of the antitumor agent of the present invention that is administered once a day are shown below in terms of a tegafur amount according to the patient's body surface area (Table 1).

Table 1

| Body surface area | Daily dose for administration once a day |
|---|---|
| Less than 1.25 m$^2$ | 80 mg, 100 mg, 120 mg, 150 mg |
| 1.25 to 1.5 m$^2$ | 100 mg, 120 mg, 150 mg, 160 mg, 180 mg |
| Not less than 1.5 m$^2$ | 120 mg, 150 mg, 160 mg, 180 mg, 200 mg |

In the present invention, as examples of "80 mg or more/dose when the patient has a body surface area of less than 1.25 m$^2$" or "100 mg or more/dose when the patient has a body surface area of less than 1.25 m$^2$," daily doses selected from the above can be mentioned. The same applies when the patient's body surface area is 1.25 to 1.5 m$^2$,

and when the patient's body surface area is not less than 1.5 m$^2$.

The blood concentration of 5-FU reaches the maximum value within 4 hours after administration, for example, about 3 hours after administration. Thereafter, the blood concentration rapidly decreases within 24 hours after administration, and 5-FU nearly disappears 24 hours after administration.

The phrase "a blood 5-FU concentration of 10 ng/ml or lower within 24 hours after administration" means that few side effects from 5-FU develop, since the concentration of 5-FU in blood nearly disappears within 24 hours after administration.

The antitumor agent of the present invention can be administered as a pharmaceutical composition. Hereinafter, the pharmaceutical composition may be referred to as "composition."

The form of the composition is not particularly limited as long as the composition is a composition for oral administration that contains tegafur, gimeracil, and oteracil potassium. Examples of the form of the composition includes tablets, coated tablets, granules, fine particles, powders, capsules, pills, emulsions, suspensions, fluids, and the like.

In the formulation of tablets, lactose, sucrose, sodium chloride, glucose, urea, starch, calcium carbonate, kaolin, crystalline cellulose, silicic acid, and other excipients; water, ethanol, propanol, simple syrup, glucose liquids, starch liquids, gelatin solutions, carboxymethyl cellulose, shellac, methyl cellulose, potassium phosphate, polyvinyl pyrrolidone, and other binders; dry starch, sodium alginate, agar powder, laminaran powder, sodium bicarbonate, calcium carbonate, polyoxyethylenesorbitan fatty acid esters, sodium lauryl sulfate, monoglyceride stearate, starch, lactose, and other disintegrators; sucrose, stearic acid, cacao butter, hydrogenated oils, and other disintegration inhibitors; quaternary-ammonium bases, sodium lauryl sulfate, and other absorbefacients; glycerin, starch, and other humectants; starch, lactose, kaolin, bentonite, colloidal silicic acid, and other adsorbents; purified talc, stearate, boric acid powder, polyethylene glycol, and other lubricants; etc., for example, may be used. Further, such tablets may be coated with typical coating materials as required, to prepare, for example, sugar-coated tablets, gelatin-coated tablets, enteric-coated tablets, film-coated tablets, double-coated tablets, multi-coated tablets, etc.

In the formulation of pills, glucose, lactose, starch, cacao butter, hydrogenated vegetable oils, kaolin, talc and other excipients; gum arabic powder, tragacanth powder, gelatin, ethanol and other binders; laminaran powder, agar powder, and other disintegrators; etc., for example, may be used.

Capsules are prepared by mixing tegafur, gimeracil, and oteracil potassium with one or more of the aforementioned various carriers; and filling hard gelatin capsules, hard capsules, or the like with the mixture.

The above-described preparations may further contain, if necessary, coloring agents, preservatives, aroma agents, flavoring agents, sweetening agents, etc.; and/or other medicines.

In the pharmaceutical composition, which contains three ingredients, i.e., tegafur, gimeracil, and oteracil potassium, as active ingredients, the proportion of tegafur, gimeracil, and oteracil potassium is usually such that, per mole of tegafur, gimeracil is used in a proportion of about 0.1 to about 5 moles and preferably about 0.1 to about 1.5 moles, and oteracil potassium is used in a proportion of about 0.1 to about 5 moles, and preferably about 0.2 to about 2 moles. It is particularly preferred that the pharmaceutical composition contains tegafur, gimeracil, and oteracil potassium in such a manner that the molar ratio of tegafur:gimeracil:oteracil potassium is 1:0.4:1. These three ingredients may be formulated into one preparation containing the three ingredients, or may be provided in a combination of different dosage forms (e.g., tablet and capsule). It is preferred that these three ingredients are formulated into one preparation containing the three ingredients.

Diseases to which the antitumor agent of the present invention can be applied are not particularly limited. Examples of the diseases include head and neck cancer, nasopharyngeal cancer, oropharyngeal cancer, hypopharyngeal cancer, pharyngeal cancer, esophageal cancer, stomach cancer, colorectal cancer, hepatocellular carcinoma, gallbladder and bile duct cancer, pancreatic cancer (cancer of head of pancreas and cancer of body of pancreas), lung cancer, breast cancer, ovarian cancer, bladder cancer, prostate cancer, renal cancer, testicular tumor, bone and soft tissue sarcomas, malignant lymphomas, leukemia, cervical cancer, skin cancer, brain tumors, and the like. Head and neck cancer, lung cancer, stomach cancer, colorectal cancer, pancreatic cancer, breast cancer, and biliary tract cancer are particularly preferred.

Cancer patients to whom the antitumor agent of the present invention is administered may be cancer patients who have not received cancer treatment, cancer patients who are currently receiving cancer treatment, or cancer patients who have ever received cancer treatment.

The antitumor agent of the present invention may be used in combination with other antitumor agents, etc., and/or radiotherapy. Examples of antitumor agents, etc., that can be used in combination with the antitumor agent of the present invention include doxorubicin, epirubicin, irinotecan hydrochloride, etoposide, docetaxel, paclitaxel, cisplatin, carboplatin, oxaliplatin, krestin, lentinan, picibanil, folinate, and levofolinate.

When the antitumor agent of the present invention is used in combination with one or more other antitumor agents, the antitumor agent of the present invention and the one or more other antitumor agents may be provided as one preparation containing the antitumor agent of the present invention, or as separate preparations. When they are provided as separate preparations, the preparations may be administered simultaneously, or one ingredient may be administered any time before or after the other ingredients are administered. The preparations are preferably administered simultaneously.

Means of administration for a composition containing the antitumor agent of the present invention and that for a composition or compositions containing the antitumor agents that are used in combination with the antitumor agent of the present invention may be the same or different (for example, oral administration and injection).

The antitumor agent of the present invention is typically administered after a meal. The time when the antitumor agent of the present invention is administered (after breakfast, after lunch, or after dinner) is not limited, as long as the antitumor agent of the present invention is administered once a day.

Examples

The present invention is described below in more detail with reference to Examples. However, the scope of the invention is not limited to these Examples.

Example 1

Human gastric cancer cell line NUGC-4 cultured on a large scale was subjected to trypsin treatment and centrifugal washing to prepare a cell suspension at a density of $1 \times 10^7$ cells/mL. The cell suspension was implanted intraperitoneally into 5- to 6-week-old male BALB/cA Jcl-nu mice using a syringe in an amount of 0.5 mL per mouse. After the body weights of the mice were measured, the mice were divided into groups with equivalent average body weights using MiSTAT ver. 1.72 group separation program. The day on which the mice were divided into the groups (n = 10) was defined as Day 0.

Test solutions of TS-1 (Taiho Pharmaceutical Co., Ltd.) (0.83 mg/mL and 1.44 mg/mL as tegafur amount) were prepared using 0.5% hydroxypropyl methylcellulose.

The test solution was orally administered to the every-day administration group at a Maximum Tolerated Dose (MTD) of 8.3 mg/kg/day from Day 1 to Day 140, every day. The test solution was orally administered to the every-other-day administration group at an MTD of 14.4 mg/kg/day from Day 1 to Day 140, every other day.

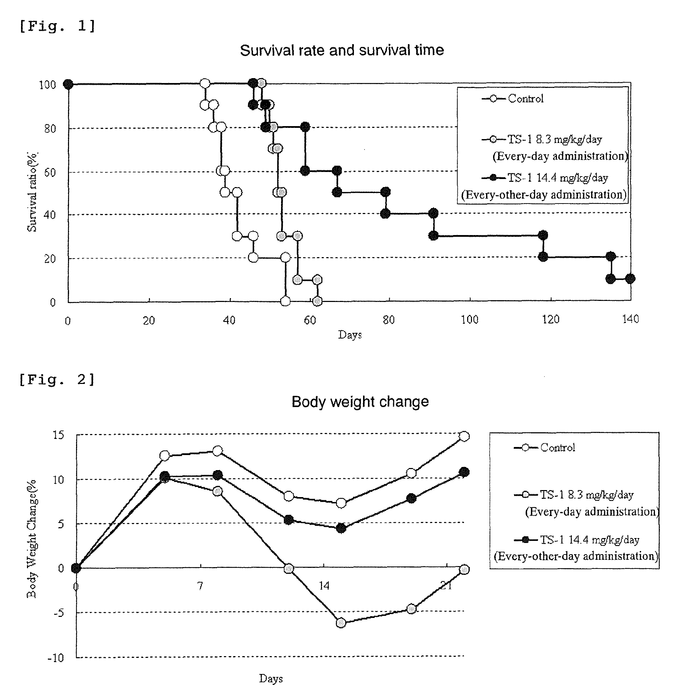

As indications of an antitumor effect, the survival rate and increase in life span (ILS(%)) were calculated by the following formulae. Fig. 1 shows the survival rates and survival times of each group. The survival time of mice that were alive on Day 140 was regarded as 140 days.

```
Survival rate (%) = (number of surviving mice/number of mice used
for evaluation) × 100
```

```
ILS (%) = [(mean survival time of drug-administration
group)/(mean survival time of control group)-1] × 100
```

The ILS of the TS-1 every-day administration group was 26.5%, and the ILS of the TS-1 every-other-day administration group was 99.3%. The administration of TS-1 significantly prolonged the survival times of the TS-1 every-day administration group and the TS-1 every-other-day administration group compared to that of the control group (p<0.01). Further, the survival times of the TS-1 every-other-day administration group were also significantly prolonged compared to those of the TS-1 every-day administration group (p<0.05). More specifically, as shown in Fig. 1, it was revealed that administering TS-1 every other day exhibits an excellent antitumor effect.

As an indication of toxicities, body weight change was measured over time. The mean body weight change (BWC(%)) of body weight on Day n to body weight on Day 0 was calculated from the following formula (body weight; BW), and is shown in Fig. 2. Fig. 2 shows the results from Day 1 to Day 22, in which the influence of cancer cells is small.

```
BWC (%) = [(BW on day n) - (BW on day 0)]/(BW on day 0) × 100
```

The dose of TS-1 in the every-other-day administration group was 14.4 mg/kg/day, which was a higher dose compared to the 8.3 mg/kg/day of the every-day administration group. Despite the higher dose, little decrease in body weight was observed in the every-other-day administration group, while severe body weight decrease accompanied by diarrhea was observed in the every-day administration group.

As described above, it was revealed that the therapeutic method using the antitumor agent of the present invention allows administration at a daily dose that is about 1.7 times the daily dose of the every-day-administration method, and is a method that exhibits an excellent antitumor effect and has low toxicities. More specifically, it was suggested that in clinical settings as well, the therapeutic method using the antitumor agent of the present invention offers high QOL and  further significantly contributes to prolongation of survival time of patients.

Example 2

TS-1 capsule (TS-1, Taiho Pharmaceutical Co., Ltd.) is repeatedly administered once a day every other day, four days a week, i.e., Monday, Wednesday, Friday, and Sunday, for 6 weeks. The 6 weeks is defined as one course, and treatment is repeated. Initial administration begins on any day (defined day) of Monday, Wednesday, Friday, and Sunday; and the day on which administration of TS-1 begins is defined as Day 1.

During Course 1, the initial dose (tegafur amount) shown in Table 2 is orally administered once a day after breakfast. When no problematic adverse events are observed after Course 1 and the doctor determines that there are no problems in regards to safety, the dose of TS-1 is increased from the initial dose according to the increased dose level shown in Table 1 from Course 2. When the dose is increased, the administration schedule is unchanged, i.e., TS-1 is orally administered once a day after breakfast on Monday, Wednesday, Friday, and Sunday.

Table 3 shows patient information, dose, continuation of administration, and the like (age and body surface area shown in the table are those at the time of initial administration).

Table 2

| Initial dose of TS-1 and increased dose level | | |
|---|---|---|
| Initial dose of TS-1 and method of increasing dose | | |
| Body surface area | Initial dose (tegafur amount) | Increased dose level |
| | | +1 |
| Less than 1.25 m$^2$ | 80 mg/day | 100 mg/day |
| Not less than 1.25 m$^2$ to less than 1.5 m$^2$ | 100 mg/day | 120 mg/day |
| Not less than 1.5 m$^2$ | 120 mg/day | 150 mg/day |

As shown in Table 4, in all of Subject Nos. 1 to 6, few hematological toxicities (decrease in leukocyte count, neutrophil count, hemoglobin count, or platelet count) and few nonhematological toxicities (onset of nausea/vomiting, loss of appetite, diarrhea, or general malaise) were also observed after Course 2, in which the dose of TS-1 was increased; and it was possible to continuously take TS-1.

In addition, a decrease in CA19-9, which is widely used as a marker for gastrointestinal cancer, and in particular as a marker for pancreatic cancer, was observed in the subjects of Subject Nos. 1, 2, 4, and 6. In particular, a significant decrease in CA19-9 was observed in the subject of Subject No. 2. These results reveal that the antitumor agent of the present invention exhibits an excellent antitumor effect. Further, since few nonhematological toxicities were observed in the subject of Subject No. 2, the subject of Subject No. 2 was capable of stable food intake. As a result, in the subject of Subject No. 2, the body weight was increased from 36.5 kg to 42.8 kg, and the body surface area was increased 1.24 m$^2$ to 1.33 m$^2$. This allowed oral administration at a tegafur amount of 120 mg. Additionally, when oral administration was performed at a tegafur amount of 120 mg, few hematological toxicities and few nonhematological toxicities were observed.

In the subjects of Subject Nos. 3 and 5, the data do not confirm an antitumor effect; however, the general health status of the patients is very good, and an excellent antitumor effect is also expected.

Further, since there are no side effects such as nausea/vomiting, loss of appetite, diarrhea, and general malaise, the antitumor agent of the present invention is also superior in QOL.

Table 3

| Subject No. | Sex (age) | Body surface area (m$^2$) | Tumor type | Course 1 From Course 2 | Administration course | Notes (complication, etc.) |
|---|---|---|---|---|---|---|
| 1 | Female (77 years old) | 1.441 | Pancreatic cancer | 100 mg<br><br>120 mg | 6 (Terminated) | Renal cancer Paraaortic lymph node metastasis |
| 2 | Female (62 years old) | 1.243 | Cancer of body of pancreas | 80 mg<br>100 mg<br>120 mg | 9 (Terminated) | Liver metastasis |
| 3 | Male (82 years old) | 1.496 | Metastatic liver cancer | 100 mg<br>120 mg | 9 (Continuing) | Liver metastasis |
| 4 | Male (69 years old) | 1.687 | Pancreatic cancer | 120 mg<br>150 mg | 9 (Terminated) | Liver metastasis |
| 5 | Male (63 years old) | 1.815 | Gallbladder cancer | 120 mg<br><br>150 mg | 5 (Terminated) | Paraaortic lymph node metastasis |
| 6 | Male (54 years old) | 1.652 | Pancreatic cancer | 120 mg<br>150 mg | 6 (Terminated) | Liver metastasis |

Table 4

| Subject No. | Test items | Before treatment | After Course 1 | After Course 2 | After Course 3 | After Course 4 | After Course 5 | After Course 6 | After Course 7 | After Course 8 | After Course 9 | Antitumor effect |
|---|---|---|---|---|---|---|---|---|---|---|---|---|
| 1 | Leukocyte count (/mm$^3$) | 5,100 | 4,770 | 3,910 | 7860 | 8840 | 5010 | 4420 | | | | CA19-9 (U/ml) |
| | Neutrophil count (/mm$^3$) | 2,930 | 2,370 | 1,935 | 6319 | 7558 | 3507 | 2652 | | | | 26,057 →23,095 →7345 (Minimum value) |
| | Hemoglobin (g/dl) | 12 | 10.6 | 9.7 | 7.5 | 9.6 | 9.7 | 8.0 | | | | |
| | Platelet count(/μl) | 289,000 | 188,000 | 277,000 | 183,000 | 145,000 | 222,000 | 216,000 | | | | |
| | Nausea/vomiting | - | - | - | - | - | - | - | | | | |
| | Loss of appetite | - | - | - | - | - | - | - | | | | |
| | Diarrhea | - | - | - | - | - | - | - | | | | |
| | General malaise | - | - | - | - | - | - | - | | | | |
| 2 | Leukocyte count (/mm$^3$) | 7,500 | 4,220 | 4,400 | 3660 | 3120 | 3120 | 3150 | 3520 | 3720 | 4070 | CA19-9 (U/ml) |
| | Neutrophil count (/mm$^3$) | 5,760 | 2,470 | 2,772 | 2170 | 1866 | 1788 | 1764 | 1478 | 1804 | 1464 | 348.2 →71.5 →15.4 |
| | Hemoglobin (g/dl) | 12.4 | 11.1 | 11.4 | 11.5 | 11.1 | 10.9 | 10.1 | 11.1 | 10.9 | 11.0 | |
| | Platelet count (/μl) | 197,000 | 149,000 | 145,000 | 140,000 | 134,000 | 131,000 | 226,000 | 234,000 | 213,000 | 251,000 | |
| | Nausea/vomiting | - | - | - | - | - | - | - | - | - | - | |
| | Loss of appetite | - | - | - | - | - | - | - | - | - | - | |
| | Diarrhea | - | - | - | - | - | - | - | - | - | - | |
| | General malaise | - | - | - | - | - | - | - | - | - | - | |

| Subject No. | Test items | Before treatment | After Course 1 | After Course 2 | After Course 3 | After Course 4 | After Course 5 | After Course 6 | After Course 7 | After Course 8 | After Course 9 | Antitumor effect |
|---|---|---|---|---|---|---|---|---|---|---|---|---|
| 3 | Leukocyte count (/mm$^3$) | 6,760 | 4,460 | 4,350 | 6350 | 6190 | 4570 | 4700 | 50.8 | 5630 | 6640 | |
| | Neutrophil count (/mm$^3$) | 4,750 | 2,410 | 2,410 | 3810 | 4340 | 2630 | 2600 | 2910 | 3690 | 4800 | |
| | Hemoglobin (g/dl) | 13.6 | 14.1 | 13.9 | 13.7 | 14.3 | 14.1 | 13.0 | 12.7 | 12.4 | 13.3 | |
| | Platelet count (/μl) | 105,000 | 140,000 | 127,000 | 123,000 | 111,000 | 107,000 | 98,000 | 99,000 | 123,000 | 102,000 | |
| | Nausea/vomiting | - | - | - | - | - | - | - | - | - | - | |
| | Loss of appetite | - | - | - | - | - | - | - | - | - | - | |
| | Diarrhea | - | - | - | - | - | - | - | - | - | - | |
| | General malaise | - | - | - | - | - | - | - | - | - | - | |
| 4 | Leukocyte count (/mm$^3$) | 7,760 | 7,710 | 6,010 | 5250 | 4620 | 4730 | 4270 | 4280 | 3800 | | CA19-9 (U/ml) |
| | Neutrophil count (/mm$^3$) | 4,940 | 4,310 | 3,020 | 2850 | 2410 | 2660 | 2410 | 2430 | 2340 | | 151.2 |
| | Hemoglobin (g/dl) | 14.8 | 13.9 | 13.5 | 12.6 | 11.5 | 11.9 | 12.3 | 11.6 | 11.0 | | →116.7 |
| | Platelet count (/μl) | 257,000 | 251,000 | 243,000 | 226,000 | 184,000 | 211,000 | 193,000 | 189,000 | 156,000 | | →93.1 |
| | Nausea/vomiting | - | - | - | - | - | - | - | - | - | | |
| | Loss of appetite | - | - | - | - | - | - | - | - | - | | |
| | Diarrhea | - | - | - | - | - | - | - | - | - | | |
| | General malaise | - | - | - | - | - | - | - | - | - | | |

EP 2 671 590 A1

| Subject No. | Test items | Before treatment | After Course 1 | After Course 2 | After Course 3 | After Course 4 | After Course 5 | After Course 6 | After Course 7 | After Course 8 | After Course 9 | Antitumor effect |
|---|---|---|---|---|---|---|---|---|---|---|---|---|
| 5 | Leukocyte count (/mm$^3$) | 6,260 | 6,060 | 5,580 | 4570 | 3600 | | | | | | |
| | Neutrophil count (/mm$^3$) | 3,512 | 3,030 | 2,960 | 2510 | 2484 | | | | | | |
| | Hemoglobin (g/dl) | 12 | 13.5 | 14.5 | 13.1 | 11.1 | | | | | | |
| | Platelet count (/μl) | 259,000 | 228,000 | 181,000 | 177,000 | 158,000 | | | | | | |
| | Nausea/vomiting | - | - | - | - | - | | | | | | |
| | Loss of appetite | - | - | - | - | - | | | | | | |
| | Diarrhea | - | - | - | - | - | | | | | | |
| | General malaise | - | - | - | - | - | | | | | | |
| 6 | Leukocyte count (/mm$^3$) | 9,610 | 5,710 | 5,570 | 4,680 | 4,420 | 4,700 | 4,010 | | | | CA19-9 (U/ml) |
| | Neutrophil count (/mm$^3$) | 8,053 | 2,840 | 3,297 | 2,320 | 2,563 | 2,350 | 2,546 | | | | 2032 |
| | Hemoglobin (g/dl) | 12.6 | 11.4 | 12.9 | 12.5 | 11.9 | 13.3 | 13.5 | | | | →138 |
| | Platelet count (/μl) | 401,000 | 283,000 | 191,000 | 165,000 | 161,000 | 173,000 | 142,000 | | | | →113.9 |
| | Nausealvomiting | - | - | - | - | - | - | - | | | | |
| | Loss of appetite | - | - | - | - | - | - | - | | | | |
| | Diarrhea | - | - | - | - | - | - | - | | | | |
| | General malaise | - | - | - | - | - | - | - | | | | |

EP 2 671 590 A1

13

Example 3

Change in blood concentration of 5-fluorouracil (5-FU) was predicted for each subject of Example 2. The concentration change was predicted from sample data (sparse sampling data) at several time points by a Bayesian estimation method according to the method described in the Journal of Pharmacokinetics and Pharmacodynamics, (2003) 30, 257-283.

Using blood concentrations of tegafur, 5-FU, and gimeracil measured 3 hours, 7 hours, and 24 hours (only some patients) after oral administration of TS-1; and sex, age, body surface area, and serum creatinine concentration at the start of a cycle of each subject, change in blood concentration of 5-FU up to 24 hours after the oral administration of TS-1 was calculated with NONMEM version VI level 2.0 (GloboMax, ICON Develop. Solutions, Ellicott City, MD, USA).

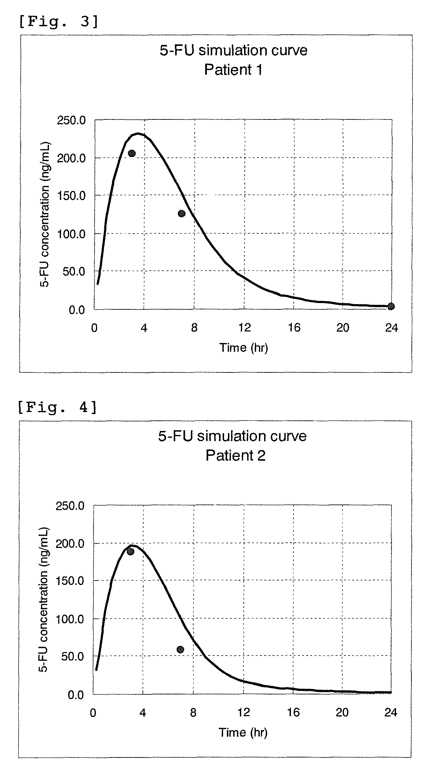

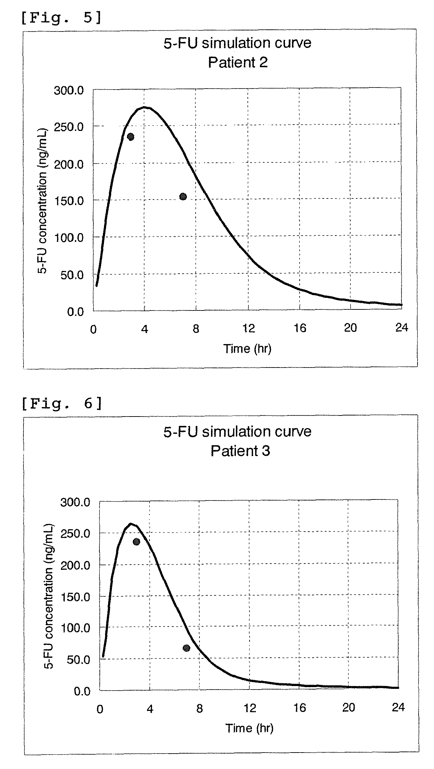

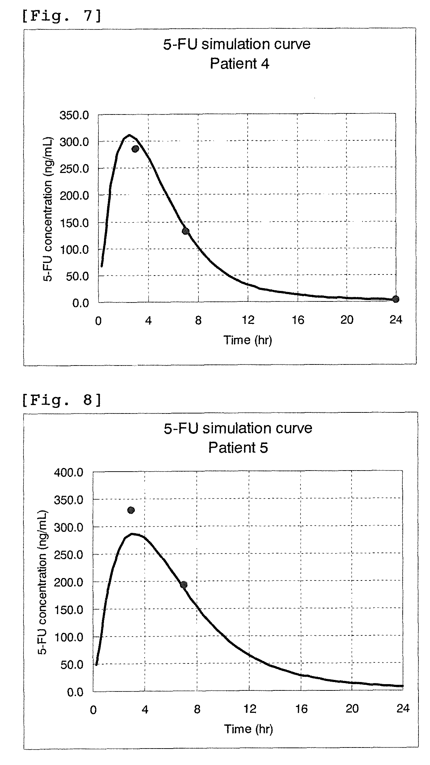

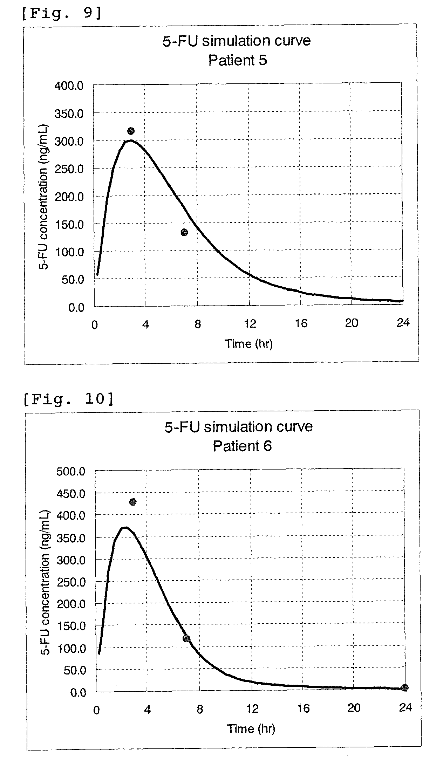

Figs. 3 to 10 show changes in blood concentrations of 5-FU of the subjects of Subject Nos. 1 to 6 at the time of Course 1. As is clear from Figs. 3 to 10, the maximum blood concentrations of 5-FU were observed around 3 hours after the administration, and their values were 180 to 450 ng/ml. Thereafter, 5-FU in blood rapidly decreased, and nearly disappeared 24 hours after the administration.

**Claims**

1. An antitumor agent comprising a combination drug containing tegafur, gimeracil, and oteracil potassium, **characterized by** being repeatedly administered to a patient once a day every other day, four days a week, at a dose such that the tegafur is administered in an amount of 80 mg or more/dose] when the patient has a body surface area of less than 1.25 $m^2$, in an amount of 100 mg or more/dose when the patient has a body surface area of 1.25 to 1.5 $m^2$, or in an amount of 120 mg or more/dose when the patient has a body surface area of not less than 1.5 $m^2$.

2. An antitumor agent comprising a combination drug containing tegafur, gimeracil, and oteracil potassium, **characterized by** being repeatedly administered to a patient once a day every other day, four days a week, at a dose such that the tegafur is administered in an amount of 100 mg or more/dose when the patient has a body surface area of less than 1.25 $m^2$, in an amount of 120 mg or more/dose when the patient has a body surface area of 1.25 to 1.5 $m^2$, or 150 mg or more/dose when the patient has a body surface area of not less than 1.5 $m^2$.

3. The antitumor agent comprising a combination drug containing tegafur, gimeracil, and oteracil potassium according to Claim 1 or 2, which gives the maximum blood 5-FU concentration of 180 to 450 ng/ml, and a blood 5-FU concentration of 10 ng/ml or lower within 24 hours after administration, in the patient administered the combination drug containing tegafur, gimeracil, and oteracil potassium.

4. The antitumor agent comprising a combination drug containing tegafur, gimeracil, and oteracil potassium according to any one of Claims 1 to 3, which gives the maximum blood 5-FU concentration of 180 to 450 ng/ml within 4 hours after administration and a blood 5-FU concentration of 10 ng/ml or lower within 24 hours after administration, in the patient administered the combination drug containing tegafur, gimeracil, and oteracil potassium.

5. The antitumor agent according to any one of Claims 1 to 4, wherein the molar ratio of respective active ingredients in the combination drug containing tegafur, gimeracil, and oteracil potassium, i.e., the molar ratio of tegafur:gimeracil: oteracil potassium, is 1:0.4:1.

6. The antitumor agent according to any one of Claims 1 to 5, wherein the combination drug is repeatedly administered every Monday, every Wednesday, every Friday, and every Sunday.

7. A method for treating cancer, comprising repeatedly administering a combination drug containing tegafur, gimeracil, and oteracil potassium to a patient once a day every other day, four days a week, at a dose such that the tegafur is administered in an amount of 80 mg or more/dose when the patient has a body surface area of less than 1.25 $m^2$, in an amount of 100 mg or more/dose when the patient has a body surface area of 1.25 to 1.5 $m^2$, or in an amount of 120 mg or more/dose when the patient has a body surface area of not less than 1.5 $m^2$.

8. A method for treating cancer, comprising repeatedly administering a combination drug containing tegafur, gimeracil, and oteracil potassium to a patient once a day every other day, four days a week, at a dose such that the tegafur is administered in an amount of 100 mg or more/dose when the patient has a body surface area of less than 1.25 $m^2$, in an amount of 120 mg or more/dose when the patient has a body surface area of 1.25 to 1.5 $m^2$ or in an amount of 150 mg or more/dose when the patient has a body surface area of not less than 1.5 $m^2$.

9. Use of a combination drug containing tegafur, gimeracil, and oteracil potassium for the production of a medicament for treating cancer, the combination drug being repeatedly administered to a patient once a day every other day, four days a week, at a dose such that the tegafur is administered in an amount of 80 mg or more/dose when the patient has a body surface area of less than 1.25 m$^2$, in an amount of 100 mg or more/dose when the patient has a body surface area of 1.25 to 1.5 m$^2$, or in an amount of 120 mg or more/dose when the patient has a body surface area of not less than 1.5 m$^2$.

10. Use of a combination drug containing tegafur, gimeracil, and oteracil potassium for the production of a medicament for treating cancer, the combination drug being repeatedly administered to a patient once a day every other day, four days a week, at a dose such that the tegafur is administered in an amount of 100 mg or more/dose when the patient has a body surface area of less than 1.25 m$^2$, in an amount of 120 mg or more/dose when the patient has a body surface area of 1.25 to 1.5 m$^2$, or in an amount of 150 mg or more/dose when the patient has a body surface area of not less than 1.5 m$^2$.

11. A combination drug containing tegafur, gimeracil, and oteracil potassium for use in the treatment of cancer, the combination drug being repeatedly administered to a patient once a day every other day, four days a week, at a dose such that the tegafur is administered in an amount of 80 mg or more/dose when the patient has a body surface area of less than 1.25 m$^2$, in an amount of 100 mg or more/dose when the patient has a body surface area of 1.25 to 1.5 m$^2$, or in an amount of 120 mg or more/dose when the patient has a body surface area of not less than 1.5 m$^2$.

12. A combination drug containing tegafur, gimeracil, and oteracil potassium for use in the treatment of cancer, the combination drug being repeatedly administered to a patient once a day every other day, four days a week, at a dose such that the tegafur is administered in an amount of 100 mg or more/dose when the patient has a body surface area of less than 1.25 m$^2$, in an amount of 120 mg or more/dose when the patient has a body surface area of 1.25 to 1.5 m$^2$, or in an amount of 150 mg or more/dose when the patient has a body surface area of not less than 1.5 m$^2$.

[Fig. 1]

**Survival rate and survival time**

[Fig. 2]

**Body weight change**

[Fig. 3]

5-FU simulation curve
Patient 1

[Fig. 4]

5-FU simulation curve
Patient 2

[Fig. 5]

5-FU simulation curve
Patient 2

[Fig. 6]

5-FU simulation curve
Patient 3

[Fig. 7]

5-FU simulation curve
Patient 4

[Fig. 8]

5-FU simulation curve
Patient 5

[Fig. 9]

5-FU simulation curve
Patient 5

[Fig. 10]

5-FU simulation curve
Patient 6

## INTERNATIONAL SEARCH REPORT

| | International application No. |
|---|---|
| | PCT/JP2012/051970 |

A. CLASSIFICATION OF SUBJECT MATTER
*A61K31/513*(2006.01)i, *A61K31/4412*(2006.01)i, *A61K31/53*(2006.01)i, *A61P35/00*(2006.01)i, *A61P43/00*(2006.01)i

According to International Patent Classification (IPC) or to both national classification and IPC

B. FIELDS SEARCHED

Minimum documentation searched (classification system followed by classification symbols)
A61K31/513, A61K31/4412, A61K31/53, A61P35/00, A61P43/00

Documentation searched other than minimum documentation to the extent that such documents are included in the fields searched
Jitsuyo Shinan Koho         1922-1996    Jitsuyo Shinan Toroku Koho    1996-2012
Kokai Jitsuyo Shinan Koho   1971-2012    Toroku Jitsuyo Shinan Koho    1994-2012

Electronic data base consulted during the international search (name of data base and, where practicable, search terms used)
CA/REGISTRY(STN), JSTPlus/JMEDPlus/JST7580(JDreamII)

C. DOCUMENTS CONSIDERED TO BE RELEVANT

| Category* | Citation of document, with indication, where appropriate, of the relevant passages | Relevant to claim No. |
|---|---|---|
| Y | Tetsuhiko SHIROSAKA, "S-1(TS-1) no Kaihatsu Keii to Saranaru Kaizen o Mezashite", Japan Gastroenterological Endoscopy Society Zasshi, 10 September 2010 (10.09.2010), vol.52 special extra issue, page 2180 | 1-6,9-12 |
| Y | UMIN CTR Rinsho Shiken Toroku Joho no Etsuran, [online], 01 December 2010 (01.12.2010), [retrieval date 21 February 2012 (21.02.2012)], Internet <URL:https://upload.umin.ac.jp/cgi-open-bin/ctr/ctr.cgi?function=brows&action=brows&type=summary&recptno=R000005467&language=J> | 1-6,9-12 |

| ☒ | Further documents are listed in the continuation of Box C. | ☐ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 23 February, 2012 (23.02.12) | 06 March, 2012 (06.03.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

| International application No. |
|---|
| PCT/JP2012/051970 |

C (Continuation).   DOCUMENTS CONSIDERED TO BE RELEVANT

| Category* | Citation of document, with indication, where appropriate, of the relevant passages | Relevant to claim No. |
|---|---|---|
| Y | TS-1 Tenpu Bunsho, [online], 2010 Nen 7 Gatsu, [retrieval date 21 February 2012 (21.02.2012)], Internet <URL:http://www.info.pmda.go.jp/ downfiles/ph/PDF/400107_4229101D1025_1_03.pdf> | 1-6,9-12 |
| T | Japanese Foundation for Multidisciplinary Treatment of Cancer, Rinsho Shiken JFMC43-1003, [online], [retrieval date 21 February 2012 (21. 02.2012)], Internet <URL:http://www.jfmc.or.jp/ product/prod04/report/JFMC43_1003.pdf> | 1-6,9-12 |

Form PCT/ISA/210 (continuation of second sheet) (July 2009)

**INTERNATIONAL SEARCH REPORT**

International application No.

PCT/JP2012/051970

| Box No. II | Observations where certain claims were found unsearchable (Continuation of item 2 of first sheet) |

This international search report has not been established in respect of certain claims under Article 17(2)(a) for the following reasons:

1. ☒ Claims Nos.: 7, 8
because they relate to subject matter not required to be searched by this Authority, namely:
Claims 7 and 8 pertain to methods for treatment of the human body by therapy and thus relate to a subject matter on which this International Searching Authority is not required to carry out a search.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

| Box No. III | Observations where unity of invention is lacking (Continuation of item 3 of first sheet) |

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**
☐ The additional search fees were accompanied by the applicant's protest and, where applicable, the payment of a protest fee.
☐ The additional search fees were accompanied by the applicant's protest but the applicable protest fee was not paid within the time limit specified in the invitation.
☐ No protest accompanied the payment of additional search fees.

Form PCT/ISA/210 (continuation of first sheet (2)) (July 2009)

**EP 2 671 590 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011018616 A

- JP 2614164 B

**Non-patent literature cited in the description**

- *Int J Clin Oncol,* 2008, vol. 13, 515-520
- *Int J Clin Oncol,* 2010, vol. 15, 166-171
- *The Japanese journal of gastroenterological surgery,* 2006, vol. 39 (4), 486

- *Nihon gan chiryo gakkaishi,* 2003, vol. 38 (2), 518
- *Journal of Pharmacokinetics and Pharmacodynamics,* 2003, vol. 30, 257-283